# EUROPEAN PATENT APPLICATION

(11) **EP 2 289 867 A2**
(43) Date of publication of application: **02.03.2011**
(21) Application number: 10008531.5
(22) Date of filing: 16.08.2010
(51) Int. Cl.: C07C 45/67, C07C 51/16, C07C 67/08, C07C 67/293, C07C 69/738, C07C 69/007, C07C 49/82, C07C 59/86

(54) **A process for producing 4-(4-halo-1-oxybutyl)-alpha,alpha-dimethylbenzene acetic acid or alkyl esters thereof**

(30) Priority: 19.08.2009 IN DE17212009
(71) Applicant: Jubilant Organosys Limited, Uttar Pradesh (IN)
(72) Inventor: Modi, Gelebith H., Noida-201 301, Uttar Pradesh (IN); Bhaskar, Bhuwan, Noida-201 301, Uttar Pradesh (IN); Tyagi, Anil Kumar, Noida-201 301, Uttar Pradesh (IN); Agarwal, Ashutosh, Noida-201 301, Uttar Pradesh (IN)
(74) Representative: Kuhnen & Wacker

(57) **Abstract**

Disclosed herein is a process for large scale production of pure 4-(4-halo-1-oxybutyl)-α,α-dimethylbenzene acetic acid or alkyl esters thereof, wherein the process comprises of condensing (2-halo-1,1-dimethyl-ethyl)benzene with acetate salt followed by acylation with ω-halo compound, hydrolyzing and cyclizing the resultant regioisomers, subsequently oxidizing and purifying to obtain pure regioisomer, further halogenating and/or esterifying the para regioisomer to produce 4-(4-halo-1-oxybutyl)-α,α-dimethylbenzene acetic acid or their alkyl esters.

## Description

### Field of the Invention

This invention, in general, relates to a process for producing highly pure 4-(4-halo-1-oxybutyl)-α,α-dimethylbenzene acetic acid or alkyl esters thereof.

### Background of the Invention

4-(4-Halo-1-oxybutyl)-α,α-dimethylbenzene acetic acid and their alkyl esters are key intermediates for the preparation of certain piperidine derivatives which are useful as antihistamines, antiallergy agents and bronchodilators.

These antihistaminic piperidine derivatives can be described by the following formula: 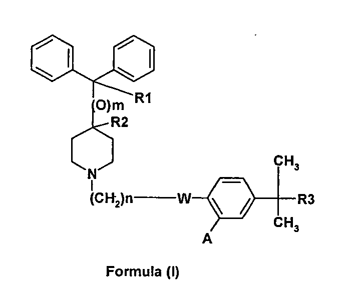 wherein the substituents are as described in US 6,348,597 viz., W represents -C(=O)- or - CH(OH)-; R₁ represents hydrogen or hydroxy; R₂ represents hydrogen; R₁ and R₂ taken together form a second bond between the carbon atoms bearing R₁ and R₂; n is an integer of from 1 to 5; m is an integer 0 or 1; R₃ is -COOH or -COOalkyl wherein the alkyl moiety has from 1 to 6 carbon atoms and is straight or branched; each of A is hydrogen or hydroxy; and pharmaceutically acceptable salts and individual optical isomers thereof, with the proviso that where R₁ and R₂ are taken together to form a second bond between the carbon atoms bearing R₁ and R₂ or where R₁ represented hydroxy, m is an integer 0.

A number of patents disclose the synthesis of these piperidine derivatives that include US 4,254,129, US 4,254,130; US 6,147,216; US 6,153,754; US 6,683,094. The process disclosed in the prior arts for producing these piperidine derivatives involves the condensation of piperidine derivatives of formula (II) with a ω-haloalkyl substituted phenyl ketone of formula (III).

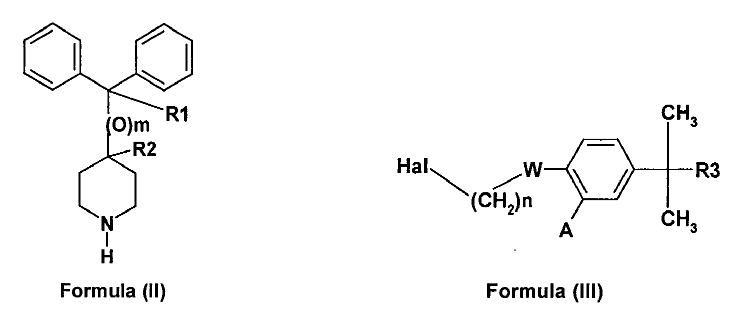

To meet the ever-increasing demand of the compound of formula (III) several processes are reported in the prior art for the production of the pure ω-haloalkyl substituted phenyl ketone.

European Patent No. 1953142 discloses a process for preparing 4-(4-chloro-1-oxybutyl)-α,α-dimethylbenzene acetic acid alkyl ester starting from cumene. The process involves reacting cumene with 4-chlorobutyryl chloride, in the presence of aluminium chloride to produce ω-chloro cumylketone, further halogenating and cyanating to give a ω-chloro-cyanocumylketone compound. The ω-halo-cyanocumylketone compound is reacted with an alcohol in the presence of anhydrous acid to give a ω-halo-α-keto-α,α-dimethylphenylacetic acid imidate compound which on hydrolysis and esterification resulted in the formation of 4-chloro-α-keto-α,α -dimethylphenylacetic acid ester compound.

US 4,254,129 and 4,254,130 discloses the preparation of ω-haloalkyl substituted phenyl ketone via Friedal Craft acylation of α,α-dimethylphenyl acetic acid alkyl ester with 4-chlorobutyryl chloride. The reaction is carried out in the presence of carbon disulfide as the preferred solvent. Moreover, the reaction results in virtually inseparable mixture of monosubstituted aromatic para and meta regioisomers where unwanted meta isomer predominates to about 65%.

US 6,548,675 reported a process for preparing the intermediate ω-haloalkyl substituted phenyl ketone using toluene as the starting material. The process involves reacting toluene with 4-chlorobutyryl chloride to form 4-chloro-tolylketone, further reacting the resultant with a base to give cyclopropyl-tolylketone. The obtained cyclopropyl-tolylketone is further subjected to halogenation, cyanation, methylation, amidation and esterification to give the product 4-chloro-α-keto-α,α -dimethylphenylacetic acid ester.

US 7,498,443 discloses another alternate process for producing methyl 4-chloro-α-keto-α,α-dimethylphenylacetate by reacting 2,2-dimethyl-1-methyl-1-trimethylsilylketene acetal with 4-bromo-γ-chlorobutyrophenone in the presence of a palladium catalyst, triaryl or alkyl phosphine and zinc salt.

European Patent No. 635004 provides a process wherein α,α-dimethylphenyl acetic acid derivative is reduced with lithium aluminium hydride to corresponding alcohol derivative, further esterification and Friedal Craft acylation produce corresponding ω-haloalkyl substituted phenyl ketone derivative. However, the patent does not disclose the yield and purity of regioisomers.

US 6,242,606 also reported a process which involves electrochemical reduction of ω-halo-halocumylketone with carbon dioxide to give corresponding ω-halo-α-keto-α,α-dimethylphenylacetic acid which is further esterified. In addition, the patent discloses another process involving reaction of cumene with cyclopropanecarboxylic acid chloride in the presence of aluminum chloride to produce cyclopropyl cumylketone compound; further halogenation to form cyclopropyl halocumylketone. Subsequently, reducing the cyclopropyl halocumylketone with carbon dioxide under electrochemical conditions and etherification and chlorination to form 4-chloro-α-keto-a,a-dimethylphenylacetic acid ester.

US 5,578,610 discloses a process for producing methyl 4-(4-iodo-1-oxobutyl)-α,α-dimethylphenylacetate wherein the mixture of regioisomers ethyl 3- and 4-(4-chloro-1-oxobutyl)-α,α-dimethylphenylacetate are transformed to another mixture of para and meta regioisomers of 3 and 4-(cyclopropyl-oxo-methyl)-α,α-dimethylphenylacetic acid and subsequently fractional crystallization of the corresponding cinchonidine salt to obtain the substantially pure 4-(cyclopropyl-oxo-methyl)-α,α-dimethylphenylacetic acid. The 4-(cyclopropyl-oxo-methyl)-α,α-dimethylphenylacetic acid is then converted to 4-(4-iodo-1-oxobutyl)-α,α-dimethylphenylacetic acid with trimethylsilyl iodide and further esterified to methyl 4-(4-iodo-1-oxobutyl)-α,α-dimethylphenylacetate. This process uses cinchonidine, which is expensive and toxic. In addition several isolation steps are necessary to obtain the desired product, thereby resulting in low yield of the final product.

US 6,458,958 discloses another process for the preparation of methyl 4-(4-chloro-1-oxobutyl)-a,a-dimethylphenylacetate starting with 4-bromophenylacetic acid. The process comprises reacting 4-bromophenylacetic acid with 2-amino-2-methyl-1-propanol to obtain 4-bromo-α-(4,4-dimethylisoxazolin-2-yl) toluene (75%) which is methylated with KHMDS and methyl iodide to form pure 4-bromo-α,α-dimethyl-a-(4,4-dimethylisoxazolin-2-yl) toluene. Subsequently, the 4-bromo-α,α-dimethyl-a-(4,4-dimethylisoxazolin-2-yl) toluene is lithiated and treated with N,N-dimethylcyclopropyl carboxylic acid amide at -78°C to give 4-(cyclopropyl-oxo-methyl)-α,α-dimethyl-a-(4,4-dimethyloxozolin-2-yl) toluene which is further hydrolysed and than esterified to obtain the product methyl 4-(4-chloro-1-oxobutyl)-α,α-dimethylphenylacetate. However, the patent does not disclose the purity of the products formed. Moreover, the process involves several steps and use of expensive raw materials.

US 6,147,216 discloses a process to obtain enriched para regioisomer by high vacuum fractional distillation of methyl or ethyl ester of the mixture of isomeric acids 3 and 4-(4-chloro-1-oxobutyl)-a,a-dimethylphenylacetic acid followed by repeated fractional crystallization at low temperatures. This process is operationally tedious, inefficient, with loss of yield at each step and therefore not amenable to industrial scale production.

The processes disclosed in the prior art include multiple steps for extraction and isolation to obtain the desired derivatives. The prior art processes involves time-consuming purification process at each step, which results in wasteful material, consequently making the process costly and uneconomical. Moreover, the processes disclosed in the prior art involve use of expensive and hazardous chemicals thereby making them unamenable for large-scale production. Further, the processes can be used only for producing small batches of the desired products and the products obtained by these processes are in low yield and at higher costs, hence making the processes unsuitable for large-scale production.

In view of the foregoing discussion and increasing demand for producing 4-(4-halo-1-oxybutyl)-α, α-dimethylbenzene acetic acid or their alkyl esters of high purity and yield, there exists a need to develop a commercially and economically viable process for large scale industrial manufacturing of 4-(4-halo-1-oxybutyl)-α, α-dimethylbenzene acetic acid or alkyl esters thereof with high purity and yield. Further, the process should involve use of less toxic materials and fewer purification steps.

### Objects and Summary of the Invention

It is a principal object of the present invention to provide a process for producing 4-(4-halo-1-oxybutyl)-α,α-dimethylbenzene acetic acid or alkyl esters thereof which are used for producing piperidine derivatives employed as antihistamines, antiallergy agents and bronchodilators.

It is another object of the present invention to provide a process for producing 4-(4-halo-1-oxybutyl)-α,α-dimethylbenzene acetic acid or alkyl esters thereof, wherein the process produces highly pure and cost effective 4-(4-halo-1-oxybutyl)-α,α-dimethylbenzene acetic acid or alkyl esters thereof at industrial scale employing cost efficient and non-hazardous raw materials.

It is yet another object of the present invention, to provide a commercially viable process for producing 4-(4-halo-1-oxybutyl)-α,α-dimethylbenzene acetic acid or alkyl esters thereof, wherein the process is employing minimal reaction, extraction and purification steps and minimal quantity of solvent during the extraction process.

The above and other objects of the present invention are further attained and supported by the following embodiments described herein. However, the scope of the invention is not restricted to the described embodiments herein after.

In accordance with a preferred embodiment of the present invention, there is provided a process for producing 4-(4-halo-1-oxybutyl)-α,α-dimethylbenzene acetic acid or alkyl esters thereof of formula IV wherein the process comprising condensing a compound of formula VI with an acetate salt to form an ester of formula VII, acylating the resultant ester of formula VII with ω-halo compound of formula VIII in presence of a catalyst and an aprotic solvent to form regioisomers of formula IX, hydrolyzing and cyclizing the regioisomers of the formula IX to form regioisomers of formula X, subsequently oxidizing the regioisomers of formula X employing an oxidizing agent to obtain crude regioisomers of formula XI, purifying the resultant to obtain pure para regioisomer of formula XII, halogenating the cyclopropyl substituent of the para regioisomer of formula XII and/or esterifying the resultant to produce pure compound of the formula IV or their alkyl esters.

In accordance with another embodiment of the present invention, there is provided an improved industrial process for producing 4-(4-halo-1-oxybutyl)-α,α-dimethylbenzene acetic acid or alkyl esters thereof of formula IV, wherein the compound of formula VI is produced by alkylating a benzene with 3-halo-2-methyl-1-propene of formula V.

In accordance with still another embodiment of the present invention, wherein the pure para regioisomer 4-(4-halo-1-oxybutyl)-α,α-dimethylbenzene acetic acid or alkyl esters thereof of formula IV produced by the process of the present invention is characterized by having purity of more than 97%.

### Detailed Description of the Invention

While the specification concludes with claims particularly pointing out and distinctly claiming that, which is regarded as the invention, it is anticipated that the invention can be more readily understood through reading the following detailed description of the invention and study of the included examples.

The present invention provides a large scale process for producing 4-(4-halo-1-oxybutyl)-α,α-dimethylbenzene acetic acid or alkyl esters thereof of formula IV. In addition the process of the present invention involves cost effective consumption of solvents, minimum generation of effluents and elimination of undesired processing steps, thereby making the process commercially viable and feasible for large scale manufacture of pure 4-(4-halo-1-oxybutyl)-α,α-dimethylbenzene acetic acid or their alkyl esters of the formula IV in lesser time. Moreover, the process of the present invention employs non-hazardous and cost effective chemicals for producing the compounds of the formula IV or alkyl esters thereof.

According to the preferred embodiment of the present invention there is provided an improved process for industrial manufacturing of the 4-(4-halo-1-oxybutyl)-α,α-dimethylbenzene acetic acid or alkyl esters thereof of formula IV with high purity and yield, which is amenable to large-scale production. 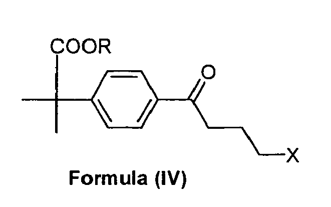 where R is H or C₁ -C₆ straight or branched alkyl group and X is Cl or Br or I.

In accordance with the present invention, the process for producing the 4-(4-halo-1-oxybutyl) -α,α-dimethylbenzene acetic acid or alkyl esters thereof of the formula IV comprises:
a) condensing a compound of formula VI with an acetate salt to form an ester of formula VII; 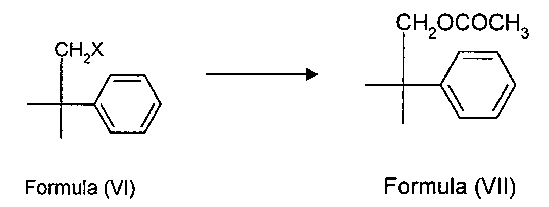 wherein, X is Cl or Br or I
b) acylating the resultant ester of formula VII with ω-halo compound of formula VIII in presence of a catalyst and an aprotic solvent to form regioisomers of formula IX; 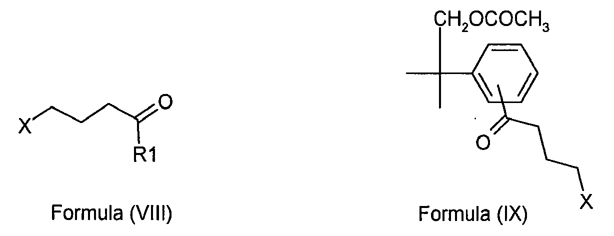 where R1 represents halo or hydroxy group and X is Cl or Br or I
c) hydrolyzing and cyclizing the regioisomers of the formula IX to form regioisomers of formula X; 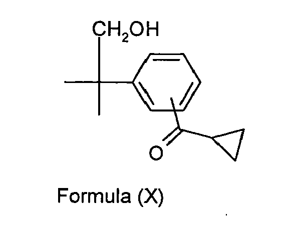
d) oxidizing the regioisomers of formula X employing an oxidizing agent to obtain crude regioisomers of formula XI and purifying the same to obtain pure para regioisomer of formula XII; and 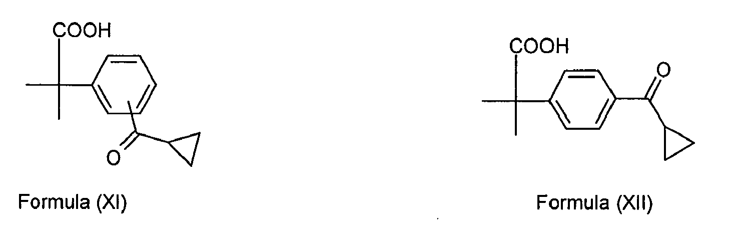
e) halogenating the cyclopropyl substituent of the para regioisomer of formula XII and/or esterifying the resultant to produce pure compound of the formula IV.

According to the present invention, the (2-halo-1,1-dimethyl-ethyl)benzene of formula VI used for the reaction is produced by Friedal Craft alkylation of a benzene with 3-halo-2-methyl-1-propene of formula V in the presence of sulphuric acid to form crude compound of the formula VI, followed by separating the crude product to obtain pure compound of the formula VI of purity > 99%. 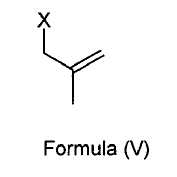 where X is as defined above.

According to the process of the present invention the condensation of the (2-halo-1,1-dimethyl-ethyl) benzene of formula VI with acetate salt is carried out in presence of a polar solvent to form corresponding crude ester of the formula VII. The undesired products obtained are separated from the reaction mass and solvent is removed to obtain pure acetic acid 2-methyl-2-phenyl-propyl ester of formula VII of purity >98%.

The acetate salt used herein the process of the present invention is selected from potassium acetate, sodium acetate or calcium acetate anhydrous or trihydrate.

The polar solvent used herein the process is selected from N-methyl pyrrolidone, sulpholan, dimethyl sulfoxide or dimethyl formamide.

According to the process of the present invention, Friedel Craft acylation of acetic acid 2-methyl-2-phenyl-propyl ester of the formula VII with the ω-halo compound of the formula VIII in the presence of a catalyst and an aprotic solvent form regioisomers of the formula IX. The undesired products obtained during the acylation reaction are separated from reaction mass by acidic washings and the pure products are isolated to obtain para and meta regioisomers of the formula IX of high yield and purity >95%.

The ω-halo compound of formula VIII used herein the process is selected from 4-chlorobutyryl chloride, 4-chlorobutyryl bromide, 4-chlorobutyryl iodide, 4-bromobutyryl chloride, 4-bromobutyric acid, 4-chlorobutyric acid, 4-iodobutyryl chloride, 4-iodobutyric acid or 4-bromobutyryl chloride.

The catalyst for acylation used herein the process is selected from any strong lewis acid aluminium chloride, titanium chloride, trifluoromethane sulfonic acid or zinc oxide, preferably aluminium chloride.

The aprotic solvent used herein the process is selected from dichloromethane, tetrahydrofuran, dimethylformamide or dimethyl sulfoxide.

According to the process of the present invention, hydrolyzing and cyclizing the regioisomers of formula IX with methanol in the presence of base form crude regioisomers of formula X. The base used herein the process is selected from sodium hydroxide or potassium hydroxide.

According to the process of the present invention, the solvent is removed from the reaction mass and further extraction with a water immiscible organic solvent and isolation of the product results in the formation of pure para and meta regioisomers of the formula X of high yield and purity >95%.

According to the process of the present invention, the regioisomers of formula X are oxidized employing an oxidizing agent, preferably potassium permanganate in the presence of acetone to form crude regioisomers of the formula XI.

In accordance with the present invention, the crude regioisomers of the formula XI are purified by separating the resultant undesired products obtained from reaction mass by filtration followed by distillation of the solvent from the separated reaction mass and extraction with a water immiscible organic solvent. The solvent is removed and product crystallized to form pure regioisomer of the formula XII with high yield and purity >97%, para isomer >95%.

The water immiscible organic solvent used herein the process for the purification is selected from cyclohexane, diethyl ether, diisopropyl ether, hexane, heptane or mixtures thereof.

According to the process of the present invention 4-(4-halo-1-oxybutyl)-a,a-dimethylbenzene acetic acid or alkyl esters thereof of the formula IV are produced by subsequently halogenating and/or esterifying substantially pure regioisomer of the formula XII to produce crude product of the formula IV. The solvent is removed from the crude product followed by dilution of the resulting mass with water, extraction with water immiscible organic solvent and isolation to produce substantially pure regioisomer of the formula IV of high yield and purity >97%.

The water immiscible organic solvent used herein the process is selected from toluene, xylene, methylene chloride or ethylene chloride.

The compound 4-(4-halo-1-oxybutyl)-α,α-dimethylbenzene acetic acid or alkyl esters thereof of formula IV produced according to the present invention acts as a key intermediate in the preparation of certain antihistamines, antiallergy agents and bronchodilators.

The present invention is further illustrated below with reference to the following examples with out intending to limit the scope of the invention in any manner.

### Example - 1

### Preparation of (2-chloro-1,1-dimethyl-ethyl)-benzene

Sulphuric acid (17.1 gm) is added to benzene (247 gm) in a RB flask under stirring at room temperature followed by adding 100 gm of 3-chloro-2-methyl-1-propene. The completion of reaction is monitored by GC. After the completion of reaction the layers are separated and the organic layer washed with brine solution. The unreacted benzene is removed to obtain (2-chloro-1,1-dimethyl-ethyl)-benzene (boiling at 97-98 °C/10 mmHg). Oil weight 130gm. Yield: 70%. GC purity: 99.5%. The product is confirmed by mass spectroscopy and ¹HNMR.
¹H NMR (400MHz) in CDCl₃: δ 7.38-7.30 ppm (m, 3H, Ar-H), δ 7.16-7.08 ppm (m, 2H, Ar-H), δ 4.13 ppm (s, 2H, -CH₂Cl), δ 1.38 ppm (s, 6H, 2x CH₃). ms: m/e 168 (M⁺ +1).

### Example - 2

### Preparation of acetic acid- (2-methyl-2-phenyl-propyl ester)

To the flask N-methyl pyrrolidone and potassium acetate (125 gm) are charged and the reaction mass heated to 160-165 °C. To this reaction mass (2-chloro-1,1-dimethyl-ethyl)-benzene obtained in Example 1 is added and stirred overnight. The completion of reaction is monitored by GC. After the completion of the reaction the crude mass is cooled to room temperature, filtered and N-methyl pyrrolidone removed to obtain acetic acid-(2-methyl-2-phenyl-propyl ester). Oil weight: 118 gm. Yield: 80%. GC purity: 98.3%. The product is confirmed by mass spectroscopy and ¹HNMR.

¹H NMR (400MHz) in CDCl₃: δ 7.38-7.30 ppm (m, 3H, Ar-H), δ 7.25-7.205 ppm (m, 2H, Ar-H), δ 4.127 ppm (s, 2H, -CH₂-O), δ 2.006 ppm (s, 3H, -COCH₃), δ 1.36 ppm (s, 6H, 2 x CH₃).
ms: m/e 192 (M⁺ +1).

### Example - 3

### Preparation of 1-acetoxy-2-methyl-2-[4-(4-chlorobutyryl)phenyl]propane and 1-acetoxy-2-methyl-2-[3-(4-chlorobutyryl)phenyl]propane

Aluminum chloride (203 gm) is charged in methylene chloride (782 gm) at room temperature under inert atmosphere. The mass is cooled to 0-5 °C and 4-chlorobutyryl chloride (130 gm) is added and the resultant mass stirred for 1-2 hrs. The reaction mass is cooled to subzero temperature and acetic acid-(2-methyl-2-phenyl-propyl ester) obtained in Example 2 is added. The mass is stirred overnight and the completion of the reaction monitored by HPLC. After the completion of the reaction, the reaction mass is added to cold DM water, the layers separated and the organic layer washed with dilute hydrochloric acid solution and again with dilute sodium bicarbonate solution. The methylene chloride is removed to obtain an oily residue containing a mixture of para and meta isomers (approximately 85:15 by GC). Oil weight: 172 gm. Yield: 95%. HPLC purity: 96.1%. The product is confirmed by mass spectroscopy and ¹HNMR.

¹HNMR (400MHz) in CDCl₃: δ 7.942-7.921 ppm (d, 2H, J=8.4Hz, Ar-H), δ 7.468-7.447 ppm (d, 2H, J=8.4Hz, Ar-H), δ 4.139 ppm (s, 2H, CH₂-OCOCH₃), δ 3.182-3.147 ppm (t, 2H, J=6.8Hz, -CH₂Cl), δ 3.685-3.655 ppm (t, 2H, J=6.0Hz, -COCH₂), δ 2.24-2.19 ppm (m, 2H, - COCH₂CH₂), δ 1.988 ppm (s, 3H, -COCH₃), δ 1.386 ppm (s, 6H, 2 x CH₃).
ms: m/e 296 (M⁺ +1).

### Example - 4

### Preparation of 2-(4-cyclopropylcarbonyl)phenyl-2-methyl propanol and 2-(3-cyclopropylcarbonyl)phenyl-2-methyl propanol

In 689 gm of methanol, 70 gm of sodium hydroxide is dissolved at room temperature followed by adding the product obtained in example 3 (172 gm). The reaction mass is stirred for 2 hours, methanol removed and DM water added to the residue. The crude product is extracted with toluene and organic layer separated and washed with DM water. The solvent is removed to obtain the pure product containing a mixture of para and meta isomers (approximately 85:15 by GC). Oil weight: 119 gm. Yield: 95%. HPLC purity: 95.9%. The product is confirmed by mass spectroscopy and ¹HNMR.

¹H NMR (400 MHz, CDCl₃) at δ 7.995-7.974 (d, 2H, J=8.4Hz, Ar-H), δ 7.504-7.483 ppm (d, 2H, J=8.4Hz, Ar-H), δ 3.64 ppm (s, 2H, -CH₂OH), δ 2.688-2.649 ppm (m, 1H), δ 1.70 ppm (s, 1H, br, -CH₂OH), δ 1.36 ppm (s, 6H, 2 x CH₃), 1.240-202 ppm (m, 2H, -CH₂), δ 1.053-1.007 (m, 2H, -CH₂),
ms: m/e 216 (M⁺ +1).

### Example - 5

### Preparation of 4-(cyclopropylcarbonyl)- α, α-dimethylphenylacetic acid

The product obtained in Example 4 is dissolved in acetone (627 gm) at room temperature followed by adding DM water and acetic acid (75 gm). The mass is cooled to 0-5 °C and potassium permanganate (180 gm) added. The resultant mass is stirred overnight and subsequently the temperature is raised to room temperature and maintained for 5hrs under stirring. The completion of the reaction is monitored by HPLC. Following completion of the reaction, the reaction mass is filtered and washed with acetone. The solvent is distilled out and sodium metabisulphite (11gm) is charged and the pH adjusted to acidic using aqueous hydrochloric acid. Thereafter, the product is extracted with methylene chloride and the solvent distilled out to obtain crude product. The crude product is then dissolved in cyclohexane in hot condition under stirring, cooled to room temperature, filtered, washed and dried to yield the pure product (para and meta isomers in ratio approximately 95:05 by GC). Dry weight: 95gm. Yield: 75%. HPLC purity: 97.5%. The product is confirmed by mass spectroscopy and ¹HNMR.

¹H NMR (400 MHz) in CDCl₃: δ (ppm) 12.6 ppm (1H, COOH), δ 8.011-7.990 ppm (d, 2H, J=8.4, Ar-H), δ 7.519-7.498 ppm (d, 2H, J=8.4, Ar-H), δ 2.688-2.649 ppm (m, 1H, -COCH-), δ 1.64 ppm (s, 6H, 2 x CH₃), δ 1.257-1.240 ppm (m, 2H, -CH₂), δ 1.069-1.024 ppm (m, 2H, - CH₂).
Ms: m/e 233 (M⁺ +1).

### Example - 6

### Preparation of 4-(4-chloro-1-Oxobutyl)- α, α-dimethylbenzene aceticacid methyl ester

The product obtained in Example-5 is added to methanolic hydrochloric acid (350 gm) at room temperature. The solution is then stirred at 40-45°C for 7 hrs and the completion of the reaction monitored by HPLC. After the completion of the reaction, methanol is removed, the concentrated mass diluted with water and product extracted with toluene. The toluene extract is washed with sodium bicarbonate solution and toluene removed under reduced pressure to obtain 4-(4-chloro-1-oxobutyl)-α,α-dimethylbenzene acetic acid methyl ester as an oily residue (para and meta isomers in ratio approximately 95:05 by GC). Oil weight: 106 gm. Yield: 95%. HPLC purity: 97.5%. The product is confirmed by mass spectroscopy and ¹HNMR.

¹H NMR (400MHz, CDCl₃) at δ 8.011-7.990 ppm (d, 2H, J=8.4, Ar-H), δ 7.519-7.498 ppm (d, 2H, J=8.4, Ar-H), δ 3.66 ppm (s, 3H, -COOCH₃), δ 3.647-3.612 ppm (t, J=6.6Hz, 2H), δ 3.159-3.124 ppm (t, J=6.6Hz, 2H), δ 2.30 ppm (p, J=6.6Hz, 2H), δ 1.61 ppm (s, 6H, 2 x CH₃).
ms: m/e 283 (M⁺ +1).

While this invention has been described in detail with reference to certain preferred embodiments, it should be appreciated that the present invention is not limited to those precise embodiments. Rather, in view of the present disclosure, which describes the current best mode for practicing the invention, many modifications and variations would present themselves to those skilled in the art without departing from the scope and spirit of this invention.

## Claims

1. A process for producing 4-(4-halo-1-oxybutyl)-α,α-dimethylbenzene acetic acid or alkyl esters thereof of formula IV 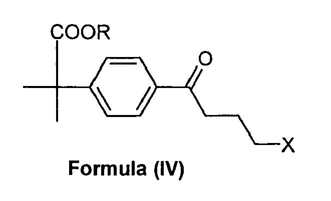 wherein R represents H or C₁ -C₆ straight or branched alkyl group and X is Cl or Br or I the process comprising:
a) condensing a compound of formula VI with an acetate salt to form an ester of formula VII; 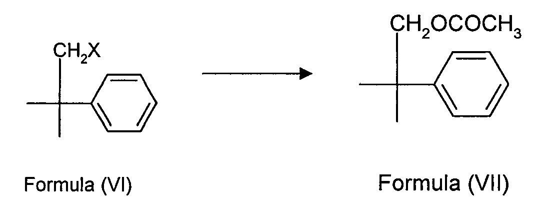 wherein, X is Cl or Br or I
b) acylating the resultant ester of the formula VII with ω-halo compound of formula VIII in presence of a catalyst and an aprotic solvent to form regioisomers of formula IX; 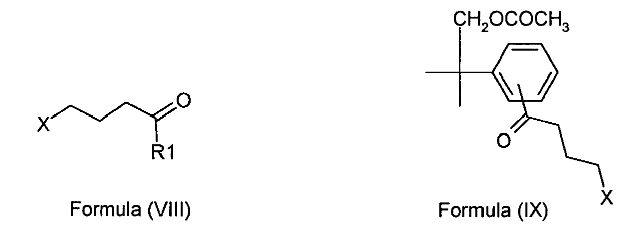 wherein R1 represents halo or hydroxy group and X is Cl or Br or I
c) hydrolyzing and cyclizing the regioisomers of the formula IX to form regioisomers of formula X; 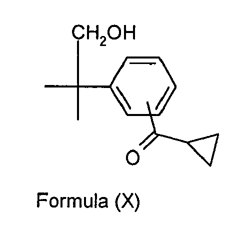
d) oxidizing the regioisomers of formula X employing an oxidizing agent to obtain crude regioisomers of formula XI and purifying the same to obtain pure para regioisomer of formula XII; and 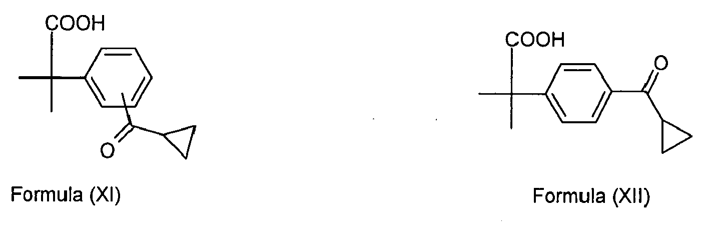
e) halogenating the cyclopropyl substituent of the para regioisomer of formula XII and/or esterifying the resultant to produce pure compound of the formula IV.

2. The process according to claim 1, wherein the compound of the formula VI is produced by alkylating a benzene with 3-halo-2-methyl-1-propene of formula V 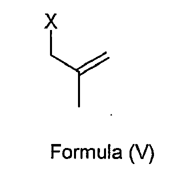 wherein X is Cl or Br or I.

3. The process according to claim 2, wherein the alkylation is carried out in presence of sulphuric acid.

4. The process according to claim 1, wherein the acetate salt used is selected from potassium acetate, sodium acetate or calcium acetate anhydrous or trihydrate.

5. The process according to claim 1, wherein the ω-halo compound of the formula VIII used is selected from 4-chlorobutyryl chloride, 4-chlorobutyryl bromide, 4-chlorobutyryl iodide, 4-bromobutyryl chloride, 4-bromobutyric acid, 4-chlorobutyric acid, 4-iodobutyryl chloride, 4-iodobutyric acid or 4-bromobutyryl chloride.

6. The process according to claim 1, wherein the catalyst used for acylating is a strong lewis acid selected from aluminium chloride, titanium (IV) chloride, trifluoromethane sulfonic acid or zinc oxide.

7. The process according to claim 6, wherein the catalyst used is aluminium chloride.

8. The process according to claim 1, wherein the aprotic solvent employed is selected from dichloromethane, tetrahydrofuran, dimethylformamide or dimethyl sulfoxide.

9. The process according to claim 1, wherein the purification of the regioisomers of the formula XI is carried out in presence of a solvent selected from water immiscible organic solvent.

10. The process according to claim 9, wherein the water immiscible organic solvent is selected from cyclohexane, diethyl ether, diisopropyl ether, hexane, heptane and mixtures thereof.

11. The process according to claim 1, wherein the condensation is carried out in presence of a polar solvent.

12. The process according to claim 11, wherein the polar solvent is selected from N-methyl pyrrolidone, sulpholan, dimethyl sulfoxide or dimethyl formamide.

13. The process according to claim 1, wherein the oxidizing agent is potassium permanganate.

14. The 4-(4-halo-1-oxybutyl)-α,α-dimethylbenzene acetic acid or esters thereof produced according to the process of claim 1 wherein the pure para regioisomer is **characterized by** having purity of more than 97%.
